# EUROPÄISCHE PATENTANMELDUNG

(11) **EP 1 652 814 A1**
(43) Veröffentlichungstag der Anmeldung: **03.05.2006**
(21) Anmeldenummer: 04025509.3
(22) Anmeldetag: 27.10.2004
(51) Int. Cl.: C01B 7/07, C01B 7/09, B01D 53/14

(54) **Verfahren zur Gastrennung**

(71) Anmelder: Solvay Fluor GmbH, 30173 Hannover (DE)
(72) Erfinder: Olschimke, Jens, 30519 Hannover (DE); Braukmüller, Saskia, 31157 Sarstedt (DE); Brosch, Carsten, 30926 Seelze (DE)
(74) Vertreter: Fischer, Reiner

(57) **Zusammenfassung**

Gasgemische, die HCl oder HBr und andere Bestandteile enthalten, insbesondere solche Gasgemische, die C(O)F₂ und HCl enthalten, können mittels ionischer Flüssigkeiten aufgetrennt werden.

## Beschreibung

Die Erfindung bezieht sich auf ein Verfahren zur Trennung von Gemischen, die HCl oder HBr und andere Bestandteile enthalten, insbesondere solche Gemische, die C(O)F₂ und HCl enthalten.

Carbonylfluorid ist als neues Ätzgas in der Halbleitertechnik und für die Reinigung von CVD-Reaktoren vorgeschlagen worden. Die nicht vorveröffentlichte Europäische Patentanmeldung 04005421.5 beschreibt ein photochemisches Verfahren zur Herstellung von C(O)F₂ aus CHClF₂. Das dort beschriebene Verfahren sieht die Herstellung von C(O)F₂ durch Photooxidation von CHClF₂ mit Sauerstoff vor. Dabei wird Licht eingestrahlt, das nicht aus einer einzigen Wellenlänge besteht, sondern einen Spektralbereich aufweist, der mindestens 50 nm umfasst (d.h. der Lichtanteil mit niedrigster Wellenlänge und der Lichtanteil mit der höchsten Wellenlänge liegen mindestens 50 nm auseinander).

Der Druck im Reaktor entspricht bei dem dort beschriebenen Verfahren bevorzugt mindestens dem Umgebungsdruck, also 1 bar (abs.). Er kann auch darüber liegen. Der Druck liegt bevorzugt im Bereich von 1 bar (abs.) bis 11 bar (abs.). Die Temperatur liegt bevorzugt im Bereich von 20 bis 300 °C, besonders im Bereich von 30 bis 300 °C und insbesondere im Bereich von 30 bis 90 °C. Vorteilhaft wählt man die Bedingungen hinsichtlich Druck und Temperatur derart, dass das Reaktionsgemisch gasförmig bleibt.

Ganz besonders bevorzugt arbeitet man bei dem dort beschriebenen Verfahren drucklos. Der Begriff "drucklos" bedeutet dort, dass auf die Reaktionsmischung außer dem Umgebungsdruck (d.h. etwa 1 bar), dem Förderdruck des Sauerstoffgases (bzw. des sauerstoffhaltigen Gases, man kann z.B. Luft oder Sauerstoff/lnertgas-Gemische einsetzen) und des gegebenenfalls eingesetzten Chlors sowie dem sich gegebenenfalls ausbildenden Druck durch bei der Reaktion entstehendes Chlorwasserstoffgas kein zusätzlicher Druck einwirkt. Der Gesamtdruck im Reaktor ist dann zweckmäßig kleiner als 2 bar absolut, je nach Förderdruck sogar kleiner als 1,5 bar absolut, aber größer als der Umgebungsdruck.

Das Verfahren kann batchweise oder bevorzugt kontinuierlich durchgeführt werden. Vorzugsweise geht man so vor, dass man kontinuierlich Ausgangsmaterial (das entsprechende Edukt, Sauerstoff bzw. ein Sauerstoff enthaltendes Gas wie Luft oder reinen Sauerstoff und gegebenenfalls Chlor) in eine Durchflussapparatur einspeist und entsprechend der eingespeisten Menge kontinuierlich Reaktionsprodukt abzieht. Die durchschnittliche Verweilzeit im Reaktionsgefäß liegt vorteilhaft zwischen 0,01 und 30 Minuten, bevorzugt zwischen 0,1 bis 3 min, besonders bevorzugt zwischen 0,3 und 1,5 Minuten. Die optimale durchschnittliche Verweilzeit, die u.a. von der Art der Lampen, der Strahlungsleistung der Lampen und von geometrischen Parametern der Bestrahlungsapparatur abhängig ist, kann man durch einfache Handversuche und Analyse des Produktstroms, beispielsweise durch Gaschromatographie, ermitteln. Vorteilhaft kann es auch sein, die Reaktionsmischung beispielsweise durch geeignete Einbauten im Reaktor gut zu verwirbeln. Die optimale Verweilzeit bei batchweiser Durchführung kann in gleicher Weise ermittelt werden.

Eine Ausführungsform sieht die Photooxidation in Abwesenheit von Chlor oder anderen Radikalinitiatoren oder Aktivatoren vor. Beispielsweise kann die Bestrahlung durch Quarzglas hindurch vorgenommen werden; andere Bauteile des Reaktors, die nicht zwischen Lichtquelle und Reaktionsgemisch angeordnet sind, können natürlich aus beliebigen Bauteilen, z.B. auch aus Borsilikatglas sein, welches bestimmte Strahlungsanteile filtert (siehe unten). Als Strahler eigenen sich übliche Strahler, die beispielsweise Strahlung im Bereich von 250 bis 400 nm oder sogar bis 600 nm abgeben (das Spektrum kann auch über die untere oder obere Grenze hinausgehen).

Eine weitere, besonders bevorzugte Ausführungsform in jener Europäischen Patentanmeldung sieht die Bestrahlung in Anwesenheit von elementarem Chlor unter Bestrahlung mit Licht einer Wellenlänge von ≥ 280 nm vor, wobei pro Gewichtsteil CHClF₂ maximal 0,6 Gewichtsteile elementares Chlor im Reaktionsgemisch enthalten sind. Bevorzugt werden pro Mol CHClF₂ 1 bis 50 mol% Chlor, vorzugsweise 5 bis 20 mol-% elementares Chlor eingesetzt.

Umsatzrate, Ausbeute und Selektivität sind bei dem dort beschriebenen Verfahren besonders hoch, wenn man in Anwesenheit von elementarem Chlor umsetzt und eine aktivierende Bestrahlung mit Licht einer Wellenlänge λ ≥ 280 nm vornimmt. Frequenzen einer Wellenlänge unterhalb von 280 nm sind dann im Wesentlichen aus dem Frequenzspektrum ausgeblendet. Dies kann man dadurch bewirken, dass man Bestrahlungslampen verwendet, die nur Licht einer Wellenlänge oberhalb oder bei 280 nm abstrahlen, und/oder man verwendet Mittel, die die entsprechenden Frequenzen aus dem abgestrahlten Licht ausblenden. Beispielsweise kann man durch Glas bestrahlen, welches nur für Licht einer Wellenlänge von 280 nm oder darüber durchlässig ist, also den Strahlungsanteil mit kürzeren Wellenlängen herausfiltert. Gut geeignet dafür sind beispielsweise Borosilikatgläser. Geeignete Gläser enthalten beispielsweise 7 bis 13 % B₂O₃, 70 bis 80 % SiO₂, ferner 2 bis 7 % Al₂O₃ und 4 bis 8 % Na₂O + K₂O sowie 0 bis 5% Erdalkalimetalloxide (jeweils Gew.-%). Bekannte Marken für Borosilikatgläser sind Duran, Pyrex und Solidex.

Das Molverhältnis zwischen dem Edukt und Sauerstoff kann bei dem in der genannten Europäischen Patentanmeldung beschriebenen Verfahren in einem weiten Bereich schwanken, zweckmäßig setzt man jedoch mindestens 0,4 Mol Sauerstoff pro Mol Ausgangsverbindung ein. Besonders gute Ergebnisse werden erzielt, wenn das Molverhältnis zwischen der Ausgangsverbindung und dem Sauerstoff im Bereich von 1:0,4 bis 1:1, insbesondere 1:0,4 bis 1:0,6 liegt. Der Sauerstoff kann wie gesagt in Form von Luft eingesetzt werden. Bevorzugt setzt man den Sauerstoff in Form eines O₂/Inertgas-Gemisches, insbesondere aber als reinen Sauerstoff ein. In Bezug auf die Produktreinheit ist es wünschenswert, dass möglichst wenig Wasser bei der Umsetzung vorhanden ist (beispielsweise weniger als 30 ppm). Gewünschtenfalls kann man die Reaktanten in bekannter Weise von mitgeschlepptem Wasser befreien, z.B. mittels Molekularsieb. Die Trennung von C(O)F₂ und HCl ist beispielsweise durch Druckdestillation möglich.

Aufgrund der eng beieinander liegenden Siedepunkte von C(O)F₂ und HCl ist eine destillative Trennung allerdings aufwendig.

Über das vorstehend beschriebene Trennproblem HCl/C(O)F₂ hinaus kann es generell wünschenswert sein, HCl oder auch HBr aus Gasgemischen, die HCl bzw. HBr zusammen mit anderen Bestandteilen enthalten, abzutrennen bzw. diese Gasgemische aufzutrennen, so dass man Gasgemische oder reine Gase erhält, die an HCl bzw. HBr abgereichert sind bzw. in welchen die andere Komponente oder die anderen Komponenten angereichert sind.

Aufgabe der vorliegenden Erfindung ist es demnach, ein einfach durchzuführendes Verfahren anzugeben, mit welchem sich HCl oder HBr aus Gasgemischen abtrennen lässt, so dass ein Gasgemisch erhalten wird, welches an HCl oder HBr abgereichert ist, bzw. worin die andere Komponente oder die anderen Komponenten angereichert sind.

Eine besondere Aufgabe der vorliegenden Erfindung ist es, ein Verfahren anzugeben, mit welchem ein an HCl abgereichertes C(O)F₂ aus Gemischen erhalten werden kann, die C(O)F₂ und HCl enthalten, bzw. mit welchem das C(O)F₂ aus diesen Gemischen angereichert werden kann. Diese Aufgabe wird durch das Verfahren der vorliegenden Erfindung gelöst.

Im weitesten Sinne wird die vorliegende Aufgabe gelöst durch das erfindungsgemäße Verfahren zur Abtrennung von HCl aus Gasgemischen, die HCl und ein oder mehrere andere gasförmige Bestandteile enthalten, unter Kontaktieren dieser Gasgemische mit einer oder mehreren ionischen Flüssigkeiten, die HCI oder mindestens eine der anderen Bestandteile des Gasgemisches unterschiedlich stark sorbieren. Das Verfahren eignet sich beispielsweise für Gasgemische, die HCl und Trifluoracetylchlorid (TFAC) enthalten. Es ist auch anwendbar auf Gasgemische, die HBr und andere Bestandteile enthalten.

Die oben genannte besondere Aufgabe zur Behandlung von C(O)F₂ und HCl enthaltenden Gasgemischen wird durch das erfindungsgemäße Verfahren zur Gewinnung von C(O)F₂, das an HCl abgereichert ist, bzw. an C(O)F₂ angereichert ist, gelöst. Dieses bevorzugte Verfahren sieht vor, dass man diese Gemische mit einer ionischen Flüssigkeit kontaktiert, die C(O)F₂ und HCl unterschiedlich stark sorbieren. Üblicherweise wird HCl von ionischen Flüssigkeiten stärker sorbiert, und ein an HCl abgereichertes C(O)F₂ wird gewonnen, bzw. das erhaltene Gas oder Gasgemisch ist an C(O)F₂ angereichert. Anhand dieser bevorzugten Anwendung wird die Erfindung weiter erläutert.

Unter ionischen Flüssigkeiten werden solche verstanden, wie sie von Wasserscheid und Keim in der Angewandten Chemie 2000,112, 3926-3945 definiert sind. lonische Flüssigkeiten sind beispielsweise als Lösungsmittel verwendbar. Wie in der genannten Druckschrift aufgeführt handelt es sich bei ionischen Flüssigkeiten um bei relativ niedrigen Temperaturen schmelzende Salze mit nichtmolekularem, ionischen Charakter. Sie sind bereits bei relativ niedrigen Temperaturen, z.B. < 100 °C, flüssig und dabei relativ niedrigviskos. Sie besitzen sehr gute Löslichkeiten für eine große Anzahl organischer, anorganischer und polymerer Substanzen.

Darüber hinaus sind ionische Flüssigkeiten in der Regel nicht brennbar, nicht korrosiv und wenig viskos und zeichnen sich durch einen nicht messbaren Dampfdruck aus.

Die lonen der in der vorliegenden Erfindung einsetzbaren ionischen Flüssigkeiten können eine oder mehrere positive bzw. negative Ladungen aufweisen, wobei lonen mit jeweils einer positiven und einer negativen Ladung bevorzugt sind.

lonische Flüssigkeiten, die sich zur Trennung von Substanzgemischen eignen, sind in der WO 02/074718 beschrieben. Sie basieren auf ionischen Flüssigkeiten, die Ammonium- oder Phosphoniumionen in den Kationen aufweisen. Generell wählt man die ionischen Flüssigkeiten so aus, dass sie keine chemische, zur Zersetzung führende Reaktion mit einer Komponente des aufzutrennenden Gasgemisches eingeht. Dies kann durch einfache Handversuche leicht sichergestellt werden. Sofern Bestandteile im Gasgemisch enthalten sind, die gegen Feuchtigkeit empfindlich sind, ist es zweckmäßig, Feuchtigkeit weitgehend auszuschließen, z.B. durch Trockenmittel im Reaktor, durch Spülen mit trockenem Inertgas oder ähnlichem. Das Gleiche gilt für eine etwaige Empfindlichkeit gegen Sauerstoff. Brauchbar sind auch weitere ionische Flüssigkeiten, z.B. mit Guanidiniumkationen.

Im Rahmen der vorliegenden Erfindung werden Kationen bevorzugt, die Stickstoff enthalten.

Im folgenden werden geeignete Kationen und Anionen weiter erläutert; dabei ist für den Fachmann klar, dass die jeweilige Kationen-/Anionenpaarung ein Produkt ergeben muss, das bei einer Temperatur von nicht mehr als 100°C flüssig sein muss, um eine nützliche lonische Flüssigkeit zu ergeben. Besonders Vorteilhaft sind ionische Flüssigkeiten, welche bei Umgebungstemperatur (etwa 20 °C) flüssig sind.

Phosphor enthaltende Kationen, insbesondere Phosphonium-Kationen mit vier gleichen oder unterschiedlichen Alkylgruppen, wie dem Butyl-, Octyl- oder Phenylrest, sind in der vorstehend erwähnten Publikation von Wasserscheid und Keim erwähnt.

Bevorzugt sind Stickstoff enthaltende Kationen; anhand dieser Ausführungsform wird die Erfindung weiter erläutert.
Im Prinzip können alle bekannten Kationen, die mindestens einen organischen Substituenten am Ammoniumkation aufweisen, verwendet werden. Allgemein handelt es sich um primäre, sekundäre, tertiäre und quartäre Ammoniumkationen. Die Substituenten können beispielsweise lineare oder verzweigte Alkylgruppen sein, beispielsweise mit 1 bis 12 C-Atomen. Es können gleiche oder unterschiedliche Alkylsubstituenten am Stickstoffatom vorliegen. Bei den Substituenten kann es sich auch um aromatische Reste handeln, beispielsweise um den Phenylrest, der gewünschtenfalls auch ein- oder mehrfach substituiert sein kann, beispielsweise durch eine oder mehrere C1-C3-Gruppen. Auch Arylalkylsubstituenten sind möglich, zum Beispiel der Benzylrest. Auch Guanidiniumkationen und Isouroniumkationen sind geeignete Kationen (derartige Verbindungen sind von Merck Darmstadt erhältlich). Die Substituenten an den Stickstoff können Wasserstoff, lineare oder verzweigte Alkylgruppen, sowie Arylgruppen sein. Die Substituenten an den Stickstoff-, Sauerstoff- und Schwefelatomen können lineare oder verzweigte Alkylgruppen, sowie Arylgruppen sein. Am Stickstoff kann auch Wasserstoff als Substituent vorhanden sein.

Auch cyclische gesättigte Ammoniumkationen sind brauchbar, beispielsweise die in der deutschen Offenlegungsschrift DE 10114565 genannten, gegebenenfalls substituierten mono- oder bicyclischen gesättigten Ammoniumkationen, beispielsweise Piperidinium oder durch Hydroxygruppen substituiertes Piperidinium. Auch die in dieser Offenlegungsschrift genannten Kationen von bicyclischen Aminen, insbesondere solche von 1,5-Diazabicyclo[4.3.0]non-5-en und 1,8-Diazabicyclo[5.4.0]-undec-7-en, sowie auch die durch Aminogruppen substitierten cyclischen Amine wie Dialkylaminopiperidin und Dialkylaminopiperazin (Alkyl bedeutet C1 bis C4) sind in Form der Kationen verwendbar.

Geeignete Kationen sind auch heterocyclische Verbindungen, die mindestens ein Stickstoffatom sowie gegebenenfalls ein Sauerstoff- oder Schwefelatom enthalten und in der genannten WO 02/074718 auf den Seiten 4 bis 6 erwähnt werden. Dabei handelt es sich um Kationen auf Basis der Struktur von Pyridin, Pyridazin, Pyrimidin, Pyrazin, Imidazol, 1H-Pyrazol, 3H-Pyrazol, 4H-Pyrazol, 1-Pyrazolin, 2-Pyrazolin, 3-Pyrazolin, 1-Imidazolin, 2-Imidazolin, 4-Imidazolin, Thiazol, Oxazol, 1,2,4-Triazol (positive Ladung am 2- bzw. 4-Stickstoffatom), 1,2,3-Triazol (positive Ladung am 2- bzw. 3-Stickstoffatom) und Pyrrolidin. Erläuterungen zu den Substituenten finden sich in der WO 02/074718 auf den Seiten 6 bis 13. Kationen von N-Alkylisochinolin, Alkyltriazolium, N-Alkylimidazolin sind ebenfalls brauchbar. Diese Strukturen können durch Wasserstoff substituiert sein. Ein oder mehrere Wasserstoffatome können auch substituiert sein, beispielsweise durch Alkylgruppen mit 1 bis 18 C-Atomen (C2-C18-Alkylgruppen können auch ein oder mehrere Sauerstoff- oder Schwefelatome oder Iminogruppen in der Kette enthalten), durch C6- bis C12-Aryl, C5-C12-Cycloalkyl oder einen fünf- bis sechsgliedrigen, Sauerstoff-, Stickstoff- oder Schwefelatome aufweisenden heterocyclischen Rest. Zwei der Substituenten können auch einen ungesättigten oder gesättigten oder aromatischen, gegebenenfalls durch ein oder mehrere Sauerstoff- oder Schwefelatome oder Iminogruppen unterbrochenen Ring bilden. Die genannten Substituenten können ihrerseits durch funktionelle Gruppen, Aryl, Alkyl, Aryloxy, Alkyloxy, Halogen, Heteroatome und/oder Heterocyclen substituiert sein.

Ein Substituent am Stickstoffatom, das die positive Ladung trägt, kann beispielsweise auch C1-C18-Alkylcarbonyl, C1-C18-Alkyloxycarbonyl, C5-C12-Cycloalkylcarbonyl oder C6-C12-Arylcarbonyl sein; dabei können auch diese Substituenten wieder durch funktionelle Gruppen, Aryl, Alkyl, Aryloxy, Alkyloxy, Halogen, Heteroatome und/oder Heterocyclen substituiert sein.

Kationen solcher Heterocyclen mit fünfgliedrigen oder sechsgliedrigen Ringen sind im Verfahren der vorliegenden Erfindung bevorzugt.

Besonders gut geeignet sind Imidazolium-, Imidazolinium-, Pyrazolium-, Oxatriazolium-, Thiatriazolium-, Pyridinium-, Pyradizinium-, Pyrimidinium- oder Pyrazinium-Kationen. Dabei können die Kohlenstoffatome bevorzugt durch Wasserstoff, C1- bis C12-Alkyl oder durch eine Hydroxy- oder CN-Gruppe substituiertes C2-C12-Alkyl sein. Das Stickstoffatom mit der positiven Ladung ist bevorzugt durch Acetyl, Methyl, Ethyl, Propyl oder n-Butyl substituiert. Es kann gegebenenfalls, wie etwaig im Ring vorhandene weitere Stickstoffatome, auch noch durch Wasserstoff oder C1-C12-Alkylgruppen substituiert sein. Bevorzugtes C1-C12-Alkyl ist Methyl, Ethyl, Propyl und n-Butyl.

Einsetzbar sind auch Oligo- und Polymere, die die vorstehend beschriebenen Moleküle enthalten (siehe z.B. M. Yoshizawa, W. Ogihara und H. Ohno, Polym. Adv. Technol. 13, 589-594, 2002).

Ganz besonders bevorzugte Kationen sind lmidazoliumkationen, die durch ein, zwei oder drei Substituenten mit je 1-24 Kohlenstoffatomen substituiert sind; dabei können die Substituenten ihrerseits beispielsweise durch Arylgruppen substituiert sein. Insbesondere bevorzugt sind 1,3-Dimethyl-imidazolium, 1-Ethyl-3-methyl-imidazolium, 1-Propyl-3-methyl-imidazolium, 1-Butyl-3-methyl-imidazolium, 1-Pentyl-3-methylimidazolium, 1-Hexyl-3-methyl-imidazolium, 1-Heptyl-3-methyl-imidazolium, 1-Octyl-3-methyl-imidazolium, 1-Nonyl-3-methyl-imidazolium, 1-Decyl-3-methyl-imidazolium, 1-Undecyl-3-methyl-imidazolium, 1-Dodecyl-3-methyl-imidazolium, 1-Benzyl-3-methylimidazolium, 1-Butyl-2,3-dimethyl-imidazolium.

Als Anionen sind besonders solche Anionen geeignet, die zur Ausbildung von Wasserstoffbrückenbindungen geeignet sind. Stark koordinierende Anionen wie z.B. Alkylsulfate oder Arylsulfate sind besser geeignet als schwach koordinierende Anionen wie Hexafluorophosphat, Tetrafluoroborat oder Trifluormethansulfonat. Geeignet sind Halogenide, besonders die Anionen von Sauerstoffsäuren oder deren Derivate wie Ester oder Amide. Gut geeignet sind ionische Flüssigkeiten mit folgenden Anionen: Alkylcarboxylate mit insgesamt 2 bis 8 C-Atomen, beispielsweise Acetat; durch Halogen, insbesondere durch Fluor substituierte Alkylcarboxylate, z.B. Trifluoracetat; Sulfat; Hydrogensulfat; Phosphat; Hydrogenphosphat; Dihydrogenphosphat; Alkylsulfat mit einem C1-C12-Alkylrest, z.B. Methyl-, Ethyl-, n-Propyl-, n-Butylsulfat, bis hin zum n-Octylsulfat; Alkyl- und Dialkylphosphat mit einem oder zwei C1-C12-Alkylresten, z.B. Methyl-, Dimethyl-, Ethyl-, Diethyl-, n-Propyl-, di-n-Propyl-, n-Butyl-, di-n-Butylphosphat;C1-C12-Alkylsulfonat, z.B. Methyl-, Ethyl-, n-Propyl-, n-Butylsulfat; Sulfonat mit einer durch ein oder mehrere Halogenatome, vorzugsweise durch Fluor substituierten C1-C12-Alkylgruppe, z.B. Arylsulfonat, z.B. Tosylat; Phosphonat mit einer C1-C12-Alkylgruppe, die direkt am Phosphor gebunden ist, z.B. Methyl-, Ethyl-, n-Propyl-, n-Butylphosphonat; Phosphonat mit einer C1-C12-Alkylgruppe, die durch ein oder mehrere Halogenatome, vorzugsweise Fluor, substituiert und direkt am Phosphor gebunden ist, z.B. Trifluormethylphosphonat; Ester der genannten Phosphonate mit einer C1-C12-Alkylgruppe, die gewünschtenfalls auch durch ein oder mehrere Halogenatome, vorzugsweise Fluoratome, substituiert sein kann; Imide von Bis(C1-C12-Alkyl)sulfonat, wobei die Alkylgruppen gewünschtenfalls durch ein oder mehrere Halogenatome, vorzugsweise Fluor, substituiert sein können, z.B. Bis(Trifluormethyl-sulfonyl)imid.

Besonders bevorzugt sind C1-C12-Alkylsulfate, insbesondere Methyl- und Ethylsulfat.

Ohne dass eine wissenschaftliche Erklärung abgegeben werden soll, weisen die Ergebnisse darauf hin, dass weniger die Polarität einer lonischen Flüssigkeit, als vielmehr das Vorhandensein von stark koordinierenden Anionen in einer lonischen Flüssigkeit, wie z.B. Methylsulfat und Ethylsulfat, die Aufnahme an HCl oder HBr beeinflusst. Schwach koordinierende bzw. "nichtkoordinierende" Anionen (S.H. Strauss, Chem. Rev. 1993, 93, 927-942) wie z.B. PF₆, BF₄, SO₃CF₃, bei welchen die negative Ladung stark delokalisiert ist, zeigen keinen besonders starken Effekt zur HCl- oder HBr-Absorption.

Bevorzugte ionische Flüssigkeiten sind solche mit einem E_{T}(30)-Wert von mehr als 20, bevorzugt mehr als 30, insbesondere mehr als 40. Dieser Wert ist ein Maß für die Polarität, siehe Reichardt, Solvent Effects in Organic Chemistry, Weinheim VCH, 1979, XI (Monographs in Modern Chemistry; 3) Seite 241 beschrieben.

Im erfindungsgemäßen Verfahren können ionische Flüssigkeiten eingesetzt werden, die nur eine Verbindung enthalten. Es können auch Gemische von zwei, drei oder mehr verschiedenen ionischen Flüssigkeiten eingesetzt werden. Dadurch kann man z.B. die Trenneigenschaften beeinflussen, beispielsweise die Polarität oder die Affinität zu einer abzutrennenden Verbindung; oder es kann die Viskosität oder die Temperatur beeinflusst werden, bei welcher das Gemisch fest wird.

Der Kontakt zwischen dem zu behandelnden Gasgemisch und der ionischen Flüssigkeit kann nach Verfahren durchgeführt werden, wie sie bei Flüssig-Gas-Operationen üblich sind. Beispielsweise kann man das zu behandelnde Gasgemisch durch die ionische Flüssigkeit hindurchleiten; durch geeignete Düsen, Fritten oder Mischeinrichtungen kann die Kontaktfläche vergrößert werden. Möglich ist z.B. ein Kontakt in einer Blasensäule. Gewünschtenfalls kann die ionische Flüssigkeit auch immobilisiert sein, z.B. auf einem Träger, z.B. einem keramischen Material oder inkorporiert in einem Polymer.

Der Druck ist in einem weiten Bereich variabel, er kann beispielsweise zwischen Umgebungsdruck bis hin zu 10 bar (absolut) oder auch höher liegen. Verfahrenstechnisch am einfachsten ist die Durchführung bei Umgebungsdruck oder einem leichten Überdruck, um das Gasgemisch in die ionische Flüssigkeit zu drücken.

Die Temperatur ist ebenfalls in einem weiten Bereich variabel. Zweckmäßig wählt man die Temperatur derart, dass die Viskosität im gewünschten Bereich liegt. Ein Temperaturbereich von 100 °C bis hinunter zur Verfestigungstemperatur der ionischen Flüssigkeit bzw. des Gemisches davon ist möglich. Bevorzugt liegt die Temperatur im Bereich von 10°C bis hin zu 80 °C.

Die Gasgemischbestandteile, die nicht in der ionischen Flüssigkeit zurückgehalten werden, können, nachdem sie diese verlassen haben, in üblicher Weise gewonnen werden. Ein Beispiel ist eine fraktionierte Kondensation oder eine Kondensation, an die sich gewünschtenfalls eine Tieftemperaturdestillation anschließen kann.

Die in der ionischen Flüssigkeit zurückgehaltenen Bestandteile können aus dieser in einer Rekonditionierung gewonnen werden, z.B. durch Anlegen eines Vakuums, Erwärmung, Durchleiten von Inertgasen oder ähnlichem. Durch die Abtrennung der in der ionischen Flüssigkeit zurückgehaltenen Bestandteile des Gasgemisches wird die ionische Flüssigkeit gleichzeitig regeneriert und ist für die erneute Anwendung brauchbar.

Das erfindungsgemäße Verfahren hat den Vorteil, dass HCl und HBr auf einfache Weise von anderen hydrolyseempfindlichen Bestandteilen einer Mischung abgetrennt werden können. Besonders vorteilhaft ist die Möglichkeit, HCl aus Gemischen mit C(O)F₂ abzutrennen, die herstellungsbedingt anfallen. Es wird hierbei ein deutlich besserer Trennungsgrad erreicht als z.B. in den Veröffentlichungen US3253029 und US 4092403, wo Acetonitril als Trennmittel eingesetzt wird. Des Weiteren besitzt die Rekonditionierung einer ionischen Flüssigkeit deutliche Vorteile gegenüber dem Recycling eines organischen Lösungsmittels wie z.B. Acetonitril.

Die folgenden Beispiele sollen die Erfindung weiter erläutern, ohne sie in ihrem Umfang einzuschränken.

### Beispiel 1: Abtrennung von HCl aus einem Gemisch mit C(O)F₂

### 1.1. Verwendung von 1-Ethyl-3-methyl-imidazoliumethylsulfat

Es wurde ein C(O)F₂/HCl Gasgemisch nach der eingangs erwähnten noch nicht vorveröffentlichte Europäischen Patentanmeldung 04005421.5 hergestellt.
Ein Rohgasgemisch der Zusammensetzung 45% C(O)F₂, 34% HCl, 6% O₂ und weiteren Reaktionskomponenten wurde mit einer Durchflussmenge von 0,8 mol/h unter leicht erhöhtem Druck durch eine Waschflasche mit 115 g (0.01 mol) 1-Ethyl-3-methylimidazoliumethylsulfat geleitet. Vorteilhaft für den Erhalt von hydrolyseempfindlichen Komponenten (wie z.B. C(O)F₂) eines Gasgemisches ist der Ausschluss bzw. Die Minimierung von Feuchtigkeit in der Apparatur und in der ionischen Flüssigkeit. Dies wurde durch Spülen der Apparatur mit trockenem Stickstoff und Evakuieren der ionischen Flüssigkeit erreicht.

Direkt nach Versuchsbeginn wurde hinter der Waschflasche mittels Gaschromatographie folgende Gaszusammensetzung detektiert: 67% C(O)F₂, 0,0% HCl, 9% O₂ und 24% weitere Reaktionskomponenten. Der prozentuale Anteil der anderen im Gasgemisch enthaltenen Komponenten verringerte sich beim Durchleiten durch die ionische Flüssigkeit nicht.

### 1.2. Verwendung von Acetonitril (Vergleichsbeispiel)

Die Versuchsdurchführung erfolgte wie unter 1.1. beschrieben.
Ein Rohgasgemisch der Zusammensetzung 47% C(O)F₂, 30% HCl, 9% O₂ und weiteren 14% Reaktionskomponenten wurde mit einem Feed von 0,8 mol/h durch eine Waschflasche mit 83 g (0.01 mol) Acetonitril geleitet. Direkt nach Versuchsbeginn wurde hinter der Waschflasche mittels Gaschromatographie folgende Gaszusammensetzung detektiert: 55% C(O)F₂, 12% HCl, 15% O₂ und 18% weitere Reaktionskomponenten.

### 1.3. Rekonditionierung der ionischen Flüssigkeit

Da eine Eigenschaft ionische Flüssigkeiten oder Gemische ionischer Flüssigkeiten ein nicht messbarer Dampfdruck ist, kann zu ihrer Rekonditionierung ein Vakuum von 10 mbar oder niedriger an die beladene Flüssigkeit angelegt werden.

Die mit HCl beladene ionische Flüssigkeit aus Beispiel 1.1 wurde bei einem verringertem Druck von 10 mbar für 1 h gehalten. Ein leichtes Erwärmen z.B. auf 40°C unter Rühren bei einer bei Raumtemperatur flüssigen ionischen Flüssigkeit beschleunigte die Gasaustreibung. Es wurde solange evakuiert, bis kein Gas mehr aus der ionischen Flüssigkeit entwich.

Mittels einfacher Massenbilanz der ionischen Flüssigkeit vor und nach der Gaseinleitung lässt sich leicht feststellen, ob die ionische Flüssigkeit vollständig rekonditioniert ist.

## Patentansprüche

1. Verfahren zur Abtrennung von HCl oder HBr aus Gasgemischen, die HCl oder HBr und ein oder mehrere andere gasförmige Bestandteile enthalten, unter Kontaktieren dieser Gasgemische mit einer oder mehreren ionischen Flüssigkeiten, die HCl oder HBr oder mindestens eine der anderen Bestandteile des Gasgemisches unterschiedlich stark sorbieren.

2. Verfahren nach Anspruch 1 zur Gewinnung von C(O)F₂, das an HCl abgereichert ist, aus Gemischen, die C(O)F₂ und HCl enthalten, unter Kontaktieren dieser Gemische mit einer oder mehreren ionischen Flüssigkeiten, die C(O)F₂ und HCl unterschiedlich stark sorbieren.

3. Verfahren nach Anspruch 2, **dadurch gekennzeichnet, dass** man eine oder mehrere ionische Flüssigkeiten verwendet, die HCl stärker sorbieren als C(O)F₂ oder dass man eine oder mehrere ionische Flüssigkeiten verwendet, die C(O)F₂ stärker sorbieren als HCl.

4. Verfahren nach Anspruch 1, **dadurch gekennzeichnet, dass** man eine Stickstoff enthaltende ionische Flüssigkeit verwendet.

5. Verfahren nach Anspruch 1, **dadurch gekennzeichnet, dass** man eine ionische Flüssigkeit verwendet, die ein Anion aufweist, welches einen E_{T}(30)-Wert von mehr als 20 aufweist und das zur Koordination befähigt ist.

6. Verfahren nach Anspruch 4, **dadurch gekennzeichnet, dass** das Anion ein Sulfonat, Sulfonamid oder ein Esteranion einer mehrbasigen Säure ist.

7. Verfahren nach Anspruch 6, **dadurch gekennzeichnet, dass** das Anion ein Monoalkylsulfat-Anion, Monoarylsulfat-Anion, ein verestertes Phosphat- oder Phosphonat-Anion ist.

8. Verfahren nach Anspruch 7, **dadurch gekennzeichnet, dass** das Anion ein Mono-C1-C12-Alkylsulfat ist.

9. Verfahren nach Anspruch 8, **dadurch gekennzeichnet, dass** das Kation zur Klasse der Imidazolium-, Imidazolinium-, Pyrazolium-, Oxatriazolium-, Thiatriazolium-, Pyridinium-, Pyradizinium-, Pyrimidinium- oder Pyrazinium-Verbindungen gehört.

10. Verfahren nach Anspruch 9, **dadurch gekennzeichnet, dass** das Kation ein Imidazoliumkation ist, das durch ein, zwei oder drei Substituenten mit je 1-24 Kohlenstoffatomen substituiert ist, wobei die Substituenten ihrerseits substituiert sein können, beispielsweise durch Arylgruppen wie Phenyl.

11. Verfahren nach Anspruch 1, **dadurch gekennzeichnet**, das das zu behandelnde Gemisch durch die ionische Flüssigkeit hindurchgeleitet Wird.

12. Verfahren nach Anspruch 11, **dadurch gekennzeichnet, dass** die ionische Flüssigkeit immobilisiert vorliegt.

13. Verfahren nach Anspruch 1, **dadurch gekennzeichnet, dass** man den Kontakt zwischen ionischer Flüssigkeit und dem zu trennenden Gemisch bei einer Temperatur im Bereich von 10 bis 80 °C und einem Druck von 1 bis 10 bar (abs) durchführt .

14. Verfahren nach Anspruch 1, **dadurch gekennzeichnet, dass** die in der ionischen Flüssigkeit sorbierten Bestandteile desorbiert werden, um die ionische Flüssigkeit zurückzugewinnen und/oder um die sorbierten Bestandteile zu gewinnen.

15. Verfahren nach Anspruch 2, **dadurch gekennzeichnet, dass** man Gemische behandelt, die aus der photochemischen Oxidation von CHClF₂ zum Zwecke der Herstellung von C(O)F₂ stammen.
